(19) **Europäisches Patentamt**

**European Patent Office**

**Office européen des brevets**

(11) Veröffentlichungsnummer: **0 291 577 B1**

(12) **EUROPÄISCHE PATENTSCHRIFT**

(45) Veröffentlichungstag der Patentschrift: **21.10.92**

(21) Anmeldenummer: **87118043.6**

(22) Anmeldetag: **07.12.87**

(51) Int. Cl.⁵: **A61N 1/36**, A61N 1/04, A61N 1/32

(54) Vorrichtung zur Therapie von Lymphstauungen u.dgl.

(30) Priorität: **20.05.87 DE 3716816**

(43) Veröffentlichungstag der Anmeldung:
**23.11.88 Patentblatt 88/47**

(45) Bekanntmachung des Hinweises auf die Patenterteilung:
**21.10.92 Patentblatt 92/43**

(84) Benannte Vertragsstaaten:
**AT BE CH DE FR GB IT LI NL SE**

(56) Entgegenhaltungen:
EP-A- 0 010 364     EP-A- 0 057 048
DE-A- 2 649 474     DE-A- 3 207 050
DE-U- 8 630 690     FR-A- 2 307 554
FR-A- 2 433 950

(73) Patentinhaber: **PHYSIOMED-Medizintechnik GmbH**
**Bayreuther Strasse 14**
**W-8563 Schnaittach b. Nbg.(DE)**

(72) Erfinder: **Seidl, Hans**
**Lehnbachweg 11**
**W-8450 Amberg(DE)**
Erfinder: **Walder, Wolfgang**
**Friedrich-Ebert-Strasse 21**
**W-8450 Amberg(DE)**
Erfinder: **Reinhold, Walter**
**Bayreuther Strasse 14**
**W-8563 Schnaittach b. Nbg.(DE)**

(74) Vertreter: **Schneck, Herbert, Dipl.-Phys., Dr. et al**
**Rau & Schneck Patentanwälte Königstrasse 2**
**W-8500 Nürnberg 1(DE)**

EP 0 291 577 B1

## Beschreibung

Die Erfindung richtet sich auf eine Vorrichtung gemäß dem Oberbegriff von Anspruch 1.

Derartige Vorrichtungen sind in unterschiedlichen Ausführungsformen bekannt. Sie umfassen in der Regel mindestens zwei Elektroden, welche jeweils einen möglichst geringen Übergangswiderstand zur Hautoberfläche des zu behandelnden Patienten aufweisen, wobei die therapeutische Wirkung im wesentlichen auf der Wirkung der durch die angelegten Elektroden im Gewebe erzeugten Ströme beruht. Dabei wird sowohl mit angelegten Gleichspannungen als auch mit Gleichspannungsimpulsen sowie mit Wechselspannungen gearbeitet. Eine besondere Therapieform wird durch die sogenannte Interferenzstrom-Therapie gebildet, bei welcher über zwei Paare von Elektroden sich überkreuzende Wechselströme mit geringfügig verschiedener Frequenz angelegt werden, wobei sich dann im Gewebe aus den angelegten Wechselströmen mittlerer Frequenz ein niederfrequenter Interferenzstrom ausbildet.

Zur Anlegung derartiger Spannungen bzw. zur Erzeugung der gewünschten therapeutisch wirksamen Ströme ist es bekannt, Elektroden, d.h. Körper mit hoher Leitfähigkeit, mit Hilfe von Kleb- oder Hafteinrichtungen am Körper zu befestigen. Es ist weiterhin bekannt, Elektroden an Handschuhen zu befestigen, die vom Therapeuten getragen werden, so daß auf diese Weise der jeweilige Ort des Wirksamwerdens der Elektrode einfach verändert werden kann. Dabei wird durch Ausbildung des Handschuhs als Schwamm-Handschuh und Befeuchtung desselben oder durch vergleichbare Maßnahmen dafür Sorge getragen, daß der Übergangswiderstand zwischen Elektrode und Haut möglichst gering gehalten wird, um unangenehme Sensationen an der Hautoberfläche durch vergleichsweise hohe fließende Ströme zu vermeiden. Auch die vorstehend angesprochene Interferenzstrom-Therapie zielt darauf ab, Ströme mit die Haut möglicherweise stark reizenden Frequenzen in das Gewebeinnere zu verlagern.

Aus DE-U-86 30 690 ist eine Kontakteinrichtung zur Behandlung von Lymphstauungen bekannt, die eine Kontaktoberfläche mit einem hohen Übergangswiderstand zur menschlichen Haut aufweist. Diese Kontakteinrichtung ist als Handschuh ausgebildet, in welchem zwei Elektroden eingebettet in einem leitfähigen Gel untergebracht sind.

Hiervon ausgehend liegt der Erfindung die Aufgabe zugrunde, eine Vorrichtung für eine hiervon abweichende, grundsätzlich neue Therapieform zu schaffen, bei der es weniger auf die Wirkung von im Gewebeinneren fließenden Strömen ankommt, sondern bei welcher das zu behandelnde Bindegewebe bzw. die Muskeln zu mechanischen Schwingungen angeregt werden, welche im Gegensatz zu der Erregung durch äußerlich angelegte Vibrations-Massagegeräte aber endogen im Gewebeinneren moduliert werden.

Die erfindungsgemäße Aufgabe wird gelöst, durch die Merkmale des kennzeichnendem Teils von Anspruch 1. Die Spannung der angelegten Impulsfolgen beträgt 100 bis 600 Volt. Vorzugsweise wird dabei mit Gleispannungsimpulsen gearbeitet.

Durch diese Ausgestaltung wird erreicht, daß in dem menschlichen Gewebe selbst nur Ströme äußerst geringer Stromstärke, d.h. im Mikroamperebereich fließen. Dies ist durch den hohen Übergangswiderstand der wenigstens einen Kontakteinrichtung bedingt, wobei durch die Strombegrenzungseinrichtung sichergestellt ist, daß auch bei vom Regelfall abweichenden Gegebenheiten, z.B. bei einem hohen Feuchtigkeitsgehalt der Umgebung, keine Gefahr für die zu behandelnde Person aufgrund der verwendeten relativ hohen Spannung besteht. Wegen der angestrebten geringen Stromstärken kann als Spannungsquelle auch eine Batterie oder ein Akkumulator verwendet werden, wobei die zu Impulsfolgen umgeformte Gleichspannung entsprechend hoch transformiert werden kann.

Durch die an das zu behandelnde Körperteil angelegten Impulsfolgen wird die Massagekraft auf das Gewebe periodisch entsprechend der erregenden Frequenz moduliert, wodurch eine äußerst wirksame Entstauung von Lymphflüssigkeit, von übersäuerter Muskulatur, Blutergüssen u. dgl. durch eine autonome Regeneration des Bindegewebes erzielt werden kann, d.h. die Konzentrationsanhäufung im Gewebe wird durchbrochen. Dabei scheint die große Wirksamkeit im Vergleich zu von außen angelegten Massageeinrichtungen insbesondere durch die endogene Modulation bedingt zu sein. Im Unterschied zu den vorgenannten bekannten Therapiegeräten verursacht die erfindungsgemäße Vorrichtung keinerlei Stromgefühl und ist somit auch bei äußerst sensiblen Patienten anwendbar. Für den Behandelten ist lediglich ein körpereigenes Pulsieren bzw. Vibrieren des Gewebes wahrnehmbar, gleich dem Pulsschlag des Herzens. Darüber hinaus ist bei dieser Art der Erregung zudem die Verwendung von Frequenzen möglich, welche mittels einer rein mechanischen Erregung nur schwer realisierbar wären. Es ist im Gegensatz zur Anregung mit mechanischen Vibrationsgeräten durch Anwendung entsprechender Impulsmuster die genaue Steuerung der Dynamik der Vibrationsbewegung möglich.

Nach dem derzeitigen Kenntnisstand sind die physiologisch-physikalischen Zusammenhänge, welche im einzelnen für das Zustandekommen dieses Effekts verantwortlich sind, noch nicht vollständig geklärt, wobei aber einiges dafür spricht, daß der Effekt durch eine periodische Änderung der Reibungskraft zwischen dem Handschuh des Therapeuten und dem behandelten Gewebe aufgrund einer dem Johnsen-Rahbeckeffekt ähnlichen Erscheinung zurückzuführen ist. Gemäß dem Johnsen-Rahbeckeffekt tritt beim Anlegen einer Spannung zwischen einer Halbleiterplatte und einer Metallplatte wegen nicht vollständiger, sondern nur punktueller Berührung eine Anziehungskraft auf, welche wiederum die Reibungskraft bei einer Relativbewegung beeinflußt. Entscheidend für das Auftreten dieses Effekts ist ein halbleiterartiges Verhalten einer der Platten, im vorliegenden Fall also des Handschuhmaterials, so daß zwar einerseits die Ausbildung von Ladungskonzentrationen an der Oberfläche möglich wird, andererseits aber vermieden wird, daß diese angesammelten Ladungen aufgrund einer zu guten Leitfähigkeit sofort abgeführt werden, ohne daß sich die erforderliche hohe Potentialdifferenz einstellt.

Das pulsierende elektrische Feld zwischen der Hand des Therapeuten und dem Körper des Patienten führt also zu einer pulsierenden elektrostatischen Anziehungskraft, damit zu einer pulsierenden Reibungskraft und diese wiederum letztlich zu einer pulsierenden Verformung des Gewebes, welche auch fühlbar ist.

Weiterhin haben Untersuchungen gezeigt, daß bei Verwendung eines sehr stark isolierenden Handschuhmaterials der zu beobachtende Effekt relativ gering ist und schnell abklingt. Demgegenüber wird mit einem Handschuh mit etwas geringerem Widerstand eine bessere Wirkung erzielt. Zudem können dann Handschuhe mit einer für den praktischen Gebrauch größeren Wandstärke verwendet werden, während bei hochisolierenden Materialien extrem dünnes Material erforderlich ist.

Vinyl-Kunststoffschicht ohne irgendwelche Leitfähigkeit erhöhende Zusätze weist den für die erfindungsgemäße Anwendung erforderlichen hohen Übergangswiderstand gegenüber der menschlichen Haut auf und wird andererseits den an eine derartige Oberfläche zu stellenden hygienischen Anforderungen gerecht.

Günstigerweise kann vorgesehen sein, daß die eine hochohmige Kontakteinrichtung als Handschuh ausgebildet ist, an dessen Innenseite eine mit der Schaltungsanordnung verbundene Elektrode angeordnet ist, und daß die andere Kontakteinrichtung als herkömmliche Haftelektrode ausgebildet ist.

Durch diese Anordnung wird unter Wahrung des hohen Übergangswiderstandes der einen Elektrode durch Vermeidung eines direkten Kontaktes derselben mit der Hautoberfläche, d.h. durch die Herstellung eines lediglich mittelbaren Kontakts über die Handschuhaußenseite, die Realisierung des erfindungsgemäßen Grundgedankens ermöglicht, wobei die grundsätzlichen Vorteile einer Handschuhelektrode gewahrt werden. Vorteilhafterweise wird bei dieser Ausführungsform als Handschuh ein Vinylhandschuh o. dgl. verwendet, d.h. ein Handschuh, der im Gegensatz zu herkömmlichen, schwammartig ausgebildeten Handschuhelektroden einen hohen Übergangswiderstand gewährleistet.

Eine andere erfindungsgemäße Ausführungsform sieht vor, daß die hochohmige Kontakteinrichtung und die Schaltungsanordnung in einem gemeinsamen Gehäuse untergebracht sind. Aufgrund der möglichen Verwendung einer Batterie oder eines Akkus als Spannungsquelle kann auch eine elektrische Zuleitung zu dem Gehäuse entfallen, so daß die ganze Vorrichtung als kompaktes, leicht handhabbares Handgerät ausgebildet werden kann. Hierbei kann der Therapeut mit seiner anderen Hand den Körper des Patienten berühren, um so den Kontakt zwischen der im Handgriff des Gerätes untergebrachten zweiten Kontaktstelle und dem Körper des Patienten herzustellen.

Je nachgiebiger das Gewebe und je langsamer die Handbewegung des Therapeuten ist, desto größer ist die zum Erreichen der Maximalkraft benötigte Pulsbreite. Die erforderliche Pulsbreite ist auch korreliert mit der eingestellten Frequenz, so daß die Einstellung des Tastverhältnisses sehr gute Anpassungsmöglichkeiten eröffnet.

Diese werden weiterhin dadurch verbessert, daß die Impulsform veränderbar ist. Durch eine geeignete Kombination von Tastverhältnis und Impulsform kann erreicht werden, daß die im Gewebe induzierte Verformung bei der Kompression in Bewegungsrichtung mit einer anderen Geschwindigkeit erfolgt als bei der anschließenden Entspannung entgegen der Bewegungsrichtung. Wegen der Geschwindigkeitsabhängigkeit der inneren Reibungskraft bei Flüssigkeiten kann dadurch die Richtung der Kraft, die auf die Gewebeflüssigkeit wirkt, vom Therapeuten gesteuert werden und somit gezielt Flüssigkeit verschoben werden.

In diesem Sinne kann weiterhin vorgesehen sein, daß die Impulsfolgen amplituden- und/oder frequenzmoduliert werden.

Zur Erzielung einer individuell einstellbaren, jeweils optimalen therapeutischen Wirkung ist eine Einrichtung zur Veränderung des Tastverhältnisses der Spannungsimpulse vorgesehen. Dabei wird vorteilhafterweise ausgegangen von einer variablen Grundfrequenz, die ca. 30 Hz betragen kann.

Vorteilhaft ist die Einstellung von Frequenz und Tastverhältnis. Hier wird Jedoch ein Impulseinsteller und ein Pauseneinsteller vorgesehen.

Nachfolgend wird die Erfindung anhand der Zeichnung Weiter berchriben. Diese zeigt schematische Blockschaltbilder zweier Ausführungsformen einer erfindungsgemäßen Vorrichtung.

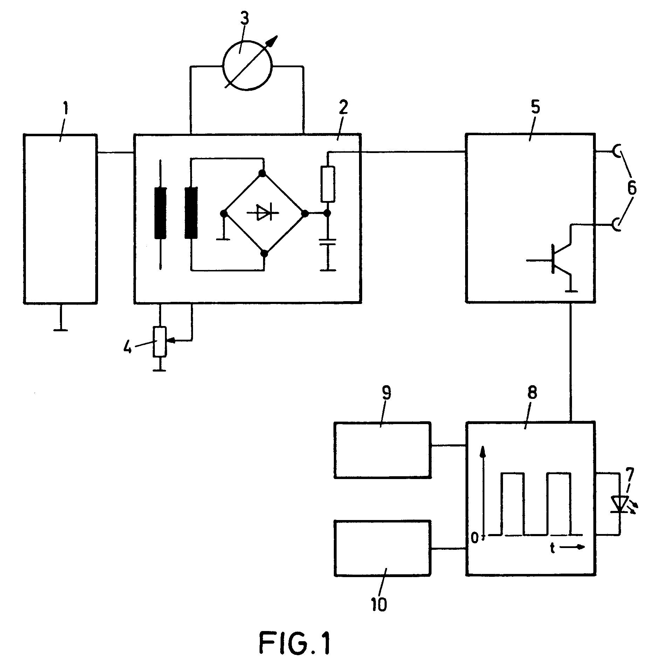

Gemäß Fig. 1 ist eine Spannungsquelle 1 in Form einer Batterie mit einem Gleichspannungs-Wandler 2 zur Erhöhung der Batteriespannung verbunden, welcher eine Strombegrenzungseinrichtung aufweist. Ein Anzeigegerät 3 zeigt die aktuelle Ausgangsspannung an.

Im Gleichspannungs-Wandler 2 ist eine Endstufe 5 mit Elektroden-Anschlußbuchsen 6 nachgeschaltet.

An die Endstufe 5 schließt sich ein Signal-Generator 8 an, welchem eine Impuls-Einstelleinrichtung 9 und eine Pausen-Einstelleinrichtung 10 zur Einstellung des Tastverhältnisses und der Impulslänge zugeordnet ist. Ein Impuls-Indikator in Form einer Leuchtdiode 7 veranschaulicht optisch die abgegebenen Impulse.

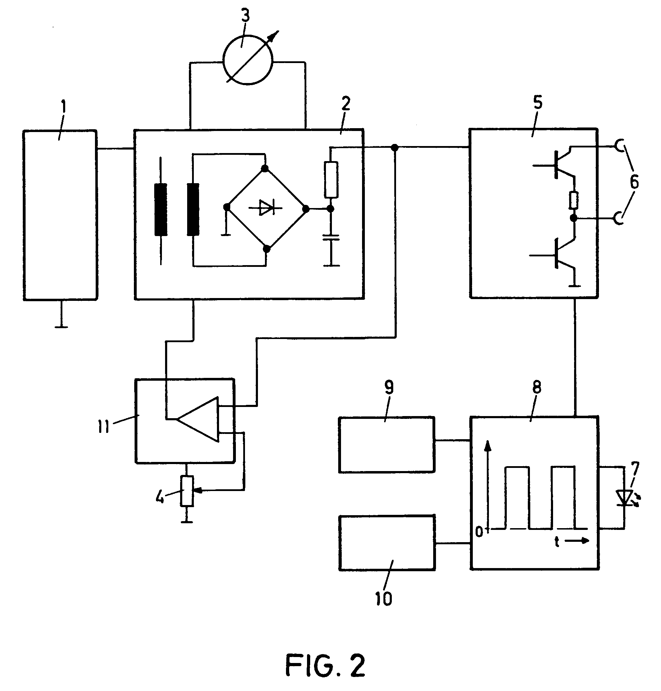

Bei der in Fig. 2 dargestellten Ausführungsform umfaßt die Endstufe 5 eine im wesentlichen durch eine Transistor-Schalter gebildete Schnellentladeschaltung, welche einerseits für eine Strombegrenzung sorgt und andererseits eine aktive Entladung des Patienten gewährleistet, was insbesondere bei Verwendung einer Folie mit sehr hohem Widerstand erforderlich ist, da dann keine Selbstentladung stattfindet.

Bei einer Folie mit geringerem Widerstand ist eine derartige aktive Entladung nicht unbedingt erforderlich, jedoch verbessert diese das Zeitverhalten, d.h. es ist die Verwendung höherer Frequenzen möglich. Zusätzlich bzw. alternativ hierzu wirkt die Verwendung von Impulsen aiternierender Polarität der Polarisierung von Folien mit hohem Widerstand entgegen.

Außerdem weist die Ausgestaltung gemäß Fig. 2 eine Anordnung 11 zur Spannungskonstanthaltung des Ausgangskreises auf, der ein Fig. 1 entsprechendes Einstellpotentiometer 4 für die Ausgangsspannung zugeordnet ist.

## Patentansprüche

1. Vorrichtung zur Therapie von insbesondere Lymphstauungen und übersäuerter Muskulatur, umfassend eine Schaltungsanordnung zur Erzeugung einer an zwei Kontakteinrichtungen anzulegenden elektrischen Spannung, wobei wenigstens eine der Kontakteinrichtungen über die Körperoberfläche beweglich ausgebildet ist und eine Kontaktoberfläche mit einem hohen Übergangswiderstand zur menschlichen Haut aufweist, dadurch gekennzeichnet, daß die Schaltungsanordnung Spannungsimpulse mit Spannungen von 100 bis 600 Volt erzeugt, der hohe Übergangswiderstand so ausgelegt ist, daß er bei Gebrauch der Vorrichtung Stromstärken im Mikroamperebereich bedingt; und die Schaltungsanordnung eine Strombegrenzungsanordnung umfaßt, die sicherstellt, daß auch bei vom Regelfall abweichenden Gegebenheiten keine Gefahr für die zu behandelnde Person aufgrund der verwendeten relativ hohen Spannung besteht.

2. Vorrichtung nach Anspruch 1, dadurch gekennzeichnet, daß die Kontaktoberfläche der Kontakteinrichtung eine Kunststoffschicht, insbesondere eine Vinylschicht, umfaßt.

3. Vorrichtung nach Anspruch 1, dadurch gekennzeichnet, daß die eine Kontakteinrichtung als Handschuh ausgebildet ist, an dessen Innenseite eine mit der Schaltungsanordnung verbundene Elektrode angeordnet ist, und daß die andere Kontakteinrichtung als herkömmliche Haftelektrode ausgebildet ist.

4. Vorrichtung nach Anspruch 1, dadurch gekennzeichnet, daß wenigstens eine der Kontakteinrichtungen und die Schaltungsanordnung in einem gemeinsamen Gehäuse untergebracht sind.

5. Vorrichtung nach Anspruch 4, dadurch gekennzeichnet, daß das Gehäuse mit einem Handgriff versehen ist.

6. Vorrichtung nach Anspruch 1, dadurch gekennzeichnet, daß die Schaltungsanordnung Gleichspannungsimpulse erzeugt und Einrichtung zur Veränderung des Tastverhältnisses umfaßt.

7. Vorrichtung nach Anspruch 1, dadurch gekennzeichnet, daß die Impulsfrequenz veränderbar ist und etwa um 30 Hz beträgt.

8. Vorrichtung nach Anspruch 1, dadurch gekennzeichnet, daß die Impulsform veränderbar ist.

9. Vorrichtung nach Anspruch 1, dadurch gekennzeichnet, daß die Impulsfolgen amplituden und/oder frequenzmoduliert werden.

10. Vorrichtung nach Anspruch 1, dadurch gekennzeichnet, daß Wechselspannungsimpulse, und eine Schaltungsanordnung mit Schnell-Entlade-Schaltung verwendet werden.

## Claims

1. Equipment for the treatment of in particular lymph blockages and hyperacidic musculature containing a switching circuit for producing an electrical voltage to be applied at two contact devices, while at least one of the contact devices is formed movably over the surface of the body and comprises one contact surface with a high transfer resistance to the human skin, characterized in that the switching circuit generates series of impulses with voltages of 100 to 600 V, that the high transfer resistance is dimensioned such that when using the device currents in the order of micro-amperes are caused; and that the switching circuit comprises a current limiting device, which assures that when conditions differ from the normal, there is no danger for the person to be treated from the relatively high voltage used.

2. Equipment according to claim 1, characterized in that the contact surface of the contact device contains a layer of synthetic material, especially a vinyl layer.

3. Equipment according to claim 1, characterized in that one contact device is shaped as a glove, on whose internal side an electrode connected with the switching circuit is provided, and in that the other contact device is formed as a conventional clamp electrode.

4. Equipment according to claim 1, characterized in that at least one of the contact devices and the switching circuit are accommodated in the same housing.

5. Equipment according to claim 4, characterized in that the housing is provided with a handle.

6. Equipment according to claim 1, characterized in that the switching circuit generates direct current impulses and contains a device to change the touching conditions.

7. Equipment according to claim 1, characterized in that the impulse frequency can be varied and is approximately 30 Hz.

8. Equipment according to claim 1, characterized in that the form of the impulse is variable.

9. Equipment according to claim 1, characterized in that the series of impulses are amplitude and/or frequency modulated.

10. Equipment according to claim 1, characterized in that alternating current impulses and a switching circuit with quick discharge circuit are used.

**Revendications**

1. Dispositif pour la thérapie, notamment de congestions lymphatiques et de musculatures hyperacidifiées, comportant un circuit destiné à produire une tension électrique à appliquer à deux dispositifs de contact, au moins un des dispositifs de contact étant conçu pour pouvoir être déplacé sur la surface du corps et comportant une surface de contact qui présente une résistance de contact élevée vis à vis de la peau humaine, caractérisé en ce que le circuit produit des impulsions de tension présentant une tension de 100 à 600 volts, en ce que la résistance de contact élevée est réalisée de manière à ce qu'elle conduit, lors de l'utilisation du dispositif, à des intensités de courant dans le domaine des micro-ampères, et en ce que le circuit comporte un dispositif de limitation du courant garantissant qu'il n'y a aucun danger pour la personne à traiter en raison des tensions relativement élevées utilisées, même dans des conditions s'écartant du cas général.

2. Dispositif selon la revendication 1, caractérisé en ce que la surface extérieure de contact du dispositif de contact, comprend une couche de matière plastique, notamment une couche de vinyle.

3. Dispositif selon la revendication 1, caractérisé en ce que l'un des dispositifs de contact est réalisé sous la forme d'un gant sur le côté intérieur duquel est disposée une électrode reliée au circuit, et en ce que l'autre dispositif de contact est réalisé en tant qu'électrode adhésive classique.

4. Dispositif selon la revendication 1, caractérisé en ce qu'au moins l'un des dispositifs de contact et le circuit sont logés dans un boîtier commun.

5. Dispositif selon la revendication 4, caractérisé en ce que le boîtier est pourvu d'une poignée.

6. Dispositif selon la revendication 1, caractérisé en ce que le circuit produit des impulsions de tension et comprend un dispositif pour faire varier le taux d'impulsions.

7. Dispositif selon la revendication 1, caractérisé en ce que la fréquence des impulsions peut être modifiée et vaut environ 30 Hz.

8. Dispositif selon la revendication 1, caractérisé en ce que la forme des impulsions peut être modifiée.

9. Dispositif selon la revendication 1, caractérisé en ce que les suites d'impulsions sont modulées en amplitude et/ou en fréquence.

10. Dispositif selon la revendication 1, caractérisé en ce que l'on utilise des impulsions de tension alternative et un circuit comportant un montage de décharge rapide.

FIG.1

FIG. 2